# EUROPEAN PATENT SPECIFICATION

(11) **EP 2 771 049 B1**
(45) Date of publication and mention of the grant of the patent: **28.06.2017**
(21) Application number: 12787832.0
(22) Date of filing: 22.10.2012
(51) Int. Cl.: A61M 5/315, A61M 5/31

(54) **A MULTI-DOSE SYRINGE WITH A CONFIGURABLE SCALE**
MEHRFACHDOSIERUNGSSPRITZE MIT KONFIGURIERBARER SKALA
SERINGUE MULTI-DOSE À GRADUATION CONFIGURABLE

(30) Priority: 24.10.2011 GB 201118290
(43) Date of publication of application: 03.09.2014
(73) Proprietor: Owen Mumford Limited, Oxford, Oxfordshire OX20 1TU (GB)
(72) Inventor: MUMFORD, Adam John, South Leigh Oxfordshire OX29 6XN (GB); EVANS, Timothy Simon, Bampton Oxfordshire OX18 2JY (GB)
(74) Representative: Lind, Robert
(86) International application number: PCT/GB2012/052613
(87) International publication number: WO 2013/061039

(56) References cited:
- EP-A1- 0 688 572
- WO-A1-01/74422
- WO-A1-2006/031227
- WO-A2-03/057285
- WO-A2-2011/067615
- US-A1- 2008 237 177

## Description

### Technical Field

The present invention relates to syringes that are capable of administering multiple doses. In particular the present invention relates to a multi-dose syringe that comprises a scale with a configurable time ordered sequence of indicia.

### Background

Pre-filled syringes that are capable of providing multiple doses are well known. They are commonly used when a person requires a regular dose of injectable medicine, for example a daily insulin injection. Syringes are marked, usually with dosage information, such that the user is able to determine the volume of medicine that has been injected. For users that require regular injections, it is not always easy to remember whether the dose for a given time period has been received. The dosage markings on the syringe typically only provide an indication of volume of medicine remaining. The user is therefore required to keep a track of when the last dose was taken in order to avoid too many doses being administered in any given time period. This is particularly problematic if the user has trouble with short-term memory, or if several people are required to administer injections to the same patient and each is possibly unaware of when a dose was last administered.

### Summary

According to a first aspect of the present invention there is provided a multi-dose injection syringe comprising a syringe body for containing a medicine and a plunger moveable within the syringe body, from an injection start position, to eject medicine therefrom, the syringe further comprising a scale provided on one of the syringe body and the plunger, and orientated along the path of progression of the plunger. The syringe is characterised in that the scale provides a time ordered sequence of indicia and in that is configurable to align any one of the indicia with said injection start position, at least part of the syringe body being transparent such that the plunger's degree of progression through the syringe body, with respect to the time ordered sequence of indicia, is externally visible.

The scale may comprise a rotatable sleeve positioned around the outside of the syringe body, the rotatable sleeve defining a window, and a number of time ordered sequences of indicia with different starting points being located on the syringe body. The scale being such that configuration may be performed by rotating the rotatable sleeve to align the window with the time ordered sequence of indicia with the required start point.

The scale may comprise a number of superposed removable labels located on the outside surface of the syringe body, each superposed label comprising a time ordered sequence of indicia with a different starting point. The scale being such that configuration may be performed by peeling off one or more of the superposed removable labels, such that the top label that remains attached to the syringe body shows the time ordered sequence of indicia with the required start point.

The scale may comprise a removable and replaceable cover that is separable into sections located around the syringe body, the cover containing the time ordered sequence of indicia. The scale being such that configuration may be performed by removing the cover from the syringe body, splitting the cover into sections at a point above the desired starting point of the time ordered sequence of indicia, and replacing the cover with the sections in a different order, such that the time ordered sequence of indicia starts at the desired starting point. The removable and replaceable cover may comprise weakening score lines above potential starting points of the time ordered sequence of indicia.

The scale may comprise a number of time ordered sequences of indicia with different starting points located on a shaped neck portion of the plunger, and a window provided in the syringe body such that one of the indicia can be viewed through the window during use of the syringe. The scale being such that configuration may be performed by selecting the time ordered sequence of indicia with the desired starting point, and lining it up with the window in the syringe prior to pressing the plunger into the syringe body. A complementary shaped opening in the syringe body may stop the shaped neck portion of the plunger from being rotated once it has been pressed into the syringe body.

The scale may comprise a number of strips removably attached along the length of the syringe body, each strip covering a time ordered sequence of indicia with different starting points located on the syringe body. The scale being such that configuration may be performed by removing the strip covering the desired time ordered sequence of indicia.

The scale may comprise a number of sleeves, each one provided with a time ordered sequence of indicia with different starting points. The scale being such that configuration may be performed by selecting one of the number of sleeves that contains the time ordered sequence of indicia with the desired starting point, and sliding the sleeve over the syringe body.

### Brief Description of the Drawings

Figures 1 and 2 show a syringe body according to a first embodiment;
Figures 3 and 4 show a syringe body according to a second embodiment;
Figure 5 shows a number of superposable labels as used in the second embodiment of figures 3 and 4;
Figures 6 and 8 show a syringe body according to a third embodiment;
Figure 7 show a removable and replaceable clip as used in the third embodiment of figures 6 and 8;
Figures 9 and 10 show a syringe according to a fourth embodiment;
Figure 11 shows apart of the syringe of Figures 9 and 10;
Figures 12 and 13 show a syringe body according to a fifth embodiment; and
Figures 14 and 15 show a syringe body according to a sixth embodiment.

### Detailed Description

As discussed above, users that use multiple dose pre-filled syringes to inject themselves or others with regular doses of medicine are required to keep track of the injection histories and requirements.

A multiple dose ("multi-dose") syringe will now be described, with reference to the accompanying figures, that comprises a scale with a configurable time ordered sequence of indicia that can present useful information to a person using the syringe regarding when a dose was last administered. The scale is formed as part of the syringe, and is configurable so as to allow the user to choose a correct starting time from the options provided within the time ordered sequence of indicia. The body of the syringe is transparent, or at least has a transparent window, such that the progress of the plunger through the body can be seen with respect to the scale provided.

The embodiments described herein and shown in the figures describe multi-dose syringes for daily injections. The time ordered sequence of indicia therefore comprises the days of the week. However, other time frames may be selected - for example, a medicine provided in a multi-dose syringe that is to be administered twice a day may have the days of the week with separate "am" and "pm" periods for each day of the week.

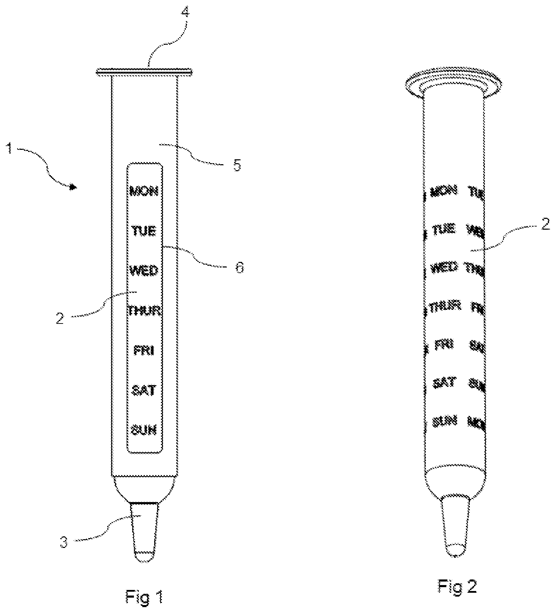

Figure 1 shows a syringe 1 comprising a syringe body 2 for containing the medicine (or a cartridge which contains the medicine), a first end 3 for connection with a hypodermic needle, and a second end 4 suitable for receiving a plunger (not shown) for pushing the medicine contained within the syringe body through the first end connected to a hypodermic needle. A rotatable sleeve 5 is provided around the body 2. The sleeve 5 defines a window 6, such that an area of the outside surface of the body 2 can be seen through the sleeve, and one of a number of areas can be selected by rotating the sleeve. The area of the outside of the surface of the syringe body 2 that is shown through the window 6 in Figure 1 contains a sequence of indicia (or "markings") that display the days of the week, starting with Monday. As a first dose is administered, the plunger will pass the Monday indication mark, and will be positioned such that it indicates that Tuesday's dose is the next one to be administered. This allows the user, or any other person, to refer to the syringe to determine if another dose is due to be administered or not. Not every course of medication will start on a Monday, so it is necessary for the user to be able to select an appropriate starting point for the markings that are provided on the syringe.

Figure 2 shows the syringe body of Figure 1 with the rotatable sleeve removed. It can be seen that the outer surface of the body 2 has a number of selectable options for which time-relevant dosage information is to be displayed through the window in the sleeve. For example, the next selectable option to the right of the one shown in Figure 1 has a starting point of Tuesday, rather than Monday. Around the outside surface of the body 2, there are seven selectable options, each one providing a sequence of indicia with a different day of the week as its starting point. Once the user has selected the sequence that is required, there may be provided a means to lock the sleeve in place, such that it does not get accidentally rotated once the course of medicine has been started.

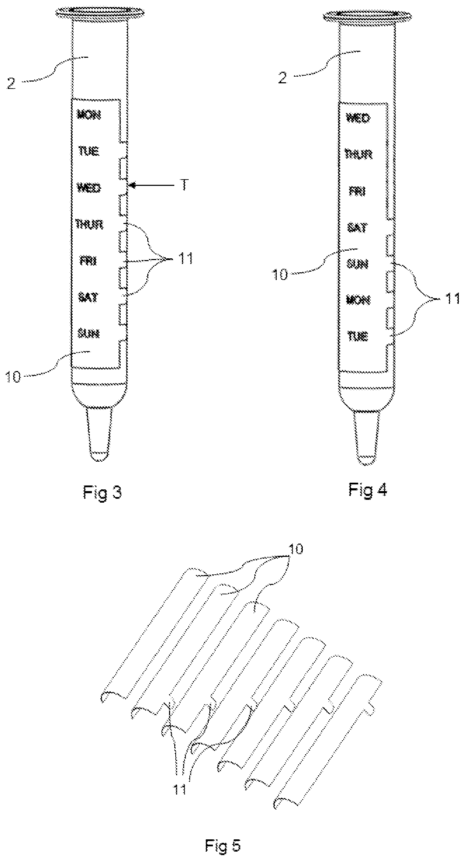

Figure 3 shows an alternative embodiment of the invention. The syringe body 2 is provided with a number of labels 10 superposed on top of each other. The top label shows the days of the week starting with Monday. Each label 10 is provided with a tab 11 that projects out of one side of the label in such a way that none of the tabs overlap with the tab of another label. The tabs 11 correspond with the markings on the top label, such that if a user selects the tab positioned next to a specific day, and removes the label to which that tab is attached (and therefore also all labels positioned on top of that label), the label that remains on the syringe body as the new top label has markings on it that show the days of the week with the day that was indicated next to the tab that was chosen as its starting point. For example, if the tab indicated by arrow T in Figure 3 is chosen and used to peel off the upper labels, the label underneath (which becomes the new top label) will show the days of the week starting with Wednesday, as shown in Figure 4. Figure 5 shows the labels that are placed on top of each other, separated out. The non-superposable tabs for each label can clearly be seen in Figure 5.

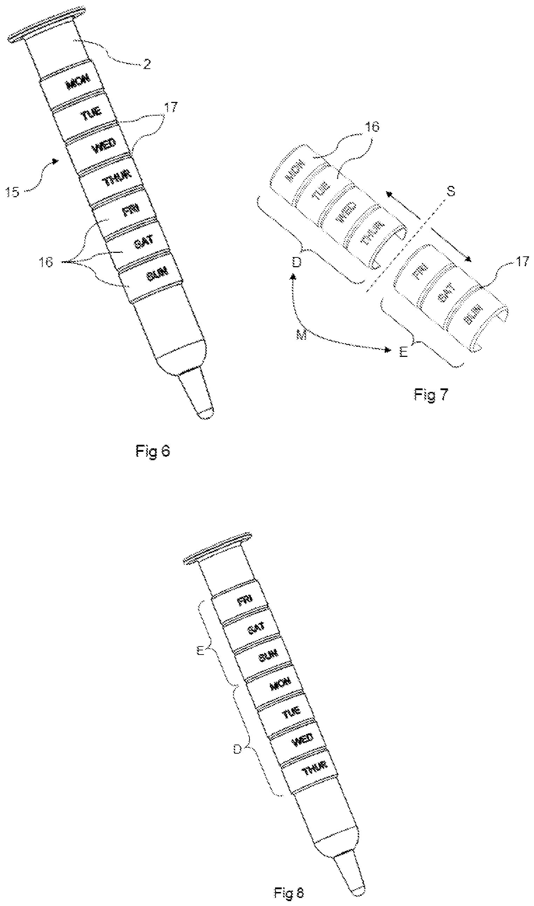

Figure 6 shows a further embodiment of the invention. The syringe body 2 is provided with a clip-on cover 15 that comprises a number of sections 16 each of which has a marking on corresponding to a day of the week. In between the sections 16, the clip-on cover 15 is provided with an area of weakness 17, for example a weakening score line, such that the clip cover can be easily broken into two or more pieces, with each piece containing one or more of the sections 16. Figure 7 shows a clip-on cover 15 that has been separated from the syringe body, and which has been broken into two pieces, D and E, at the dotted line S. The first piece D contains four sections 16 that contain markings for Monday to Thursday, and the second piece E contains 3 sections containing markings for Friday to Sunday. Now that these two pieces have been separated, the user can switch their positions as shown by arrow M, and replace them onto the syringe body in the desired order, such as shown in Figure 8. In Figure 8, the piece E containing three sections has been replaced above the second piece D. The sequence of indicia now starts with Friday at the top of the syringe instead of Monday. The clip can be separated between any of the sections, and so the user is able to configure the scale to put the required starting point (e.g. day of the week) at the top of the syringe.

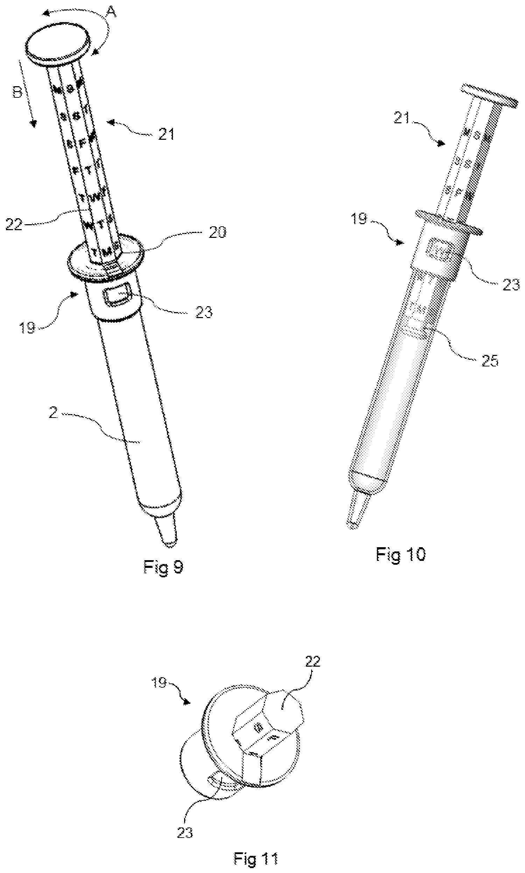

A fourth embodiment of the invention is shown in Figures 9 and 10. An end cap 19 is provided over the end of the syringe body that is suitable for receiving a plunger. The cap 19 defines a shaped opening 20 through which the plunger 21 is received. The neck 22 of the plunger has a complimentary shape such that it can fit through the opening in the end of the syringe body, but once inside the syringe body the plunger cannot be rotated. The faces provided on the neck 22 of the plunger contain the sequence of indicia. Each face provides a sequence of indicia with a different starting point. A window in the end cap 19 aligns with one of the faces, and allows one marking to be viewed by the user. As can be seen in Figure 10, the end of the plunger that is inserted into the syringe body has a portion of reduced diameter 25. This portion of reduced diameter 25, when positioned at the shaped opening 20, allows the plunger to be rotated such that the face that contains the sequence of indicia with the desired starting point can be selected. Arrow A in Figure 9 shows the selection of the desired plunger face. The correct face is selected when it is aligned with the window 23 in the end cap 19. Once it has been aligned correctly, the plunger is pressed into the syringe body, as shown by arrow B, such that the shaped neck of the plunger above the portion of reduced diameter is inserted through the complimentarily shaped opening 20. This locks the plunger such that is can no longer be rotated. The end cap 19 and a part of the shaped neck 22 of the plunger can be seen more clearly in Figure 11. The shaped neck of the plunger 22 has seven faces (the cross section of the plunger neck is a regular heptagon), which allows for seven different selectable options for markings, as similarly provided in the other embodiments of the invention.

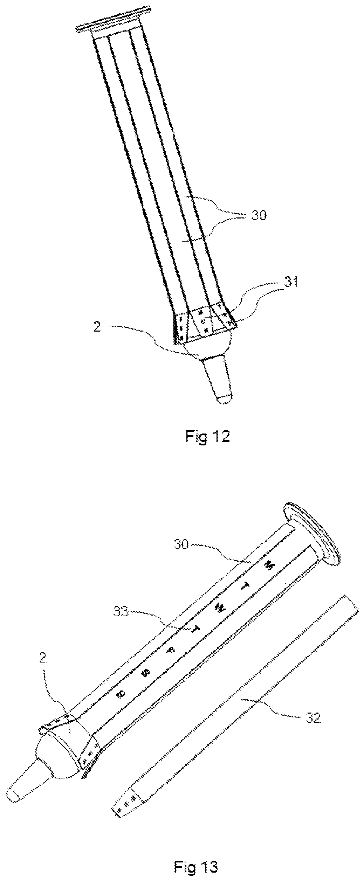

Figure 12 shows a fifth embodiment of the invention. The outside of the syringe body 2 is provided with a number of peelable strips 30 along its length. Each peelable strip 30 is provided with a tab 31 that enables the user to select one of the strips and peel it off the syringe body. Each of the tabs 31 in Figure 12, indicate a day of the week - this corresponds to the starting point for the sequence of indicia that will be selected by peeling of the strip 30 connected to that tab 31. For example, as shown in Figure 13, the strip 32, which has a tab indicating "MON" (for Monday), has been peeled off and removed from the syringe body. The outer surface 33 of the syringe body 2 that was previously hidden from view by the strip 32 can now be seen. The days of the week markings, starting with Monday, are provided on the area of the outer surface 33 that has been revealed.

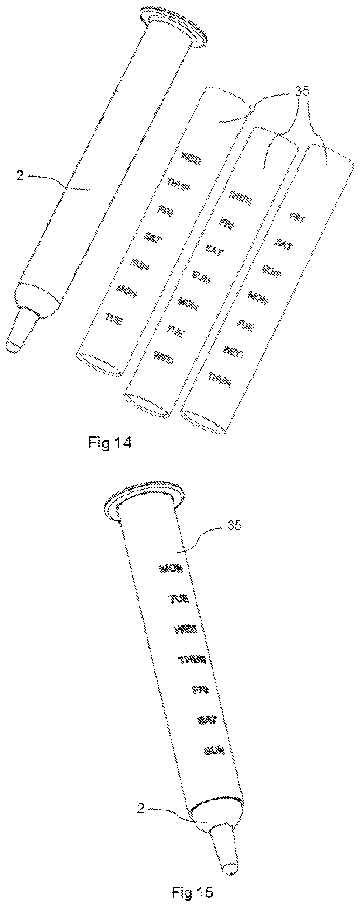

A sixth embodiment of the invention is shown in Figures 14 and 15. As shown in Figure 14, a plain syringe body 2 is provided with a number of transparent sleeves 35, each of which contain sequences of indicia with different starting points. Three sleeves 35 are shown in Figure 14, but any number of sleeves can be provided according to the requirements of the syringe use. The sleeve that contains the sequence of indicia with the deired starting point is selected and slid into place around the plain syringe body. The sleeve can be made to have a close fit with the syringe body 2, such that once it is in position around the syringe body, it is held in place by frictional force between the two. Alternatively, the syringe body may comprise a means for locking the sleeve in place.

In a further alternative, a syringe as illustrated in Figure 2 could be used together with a plunger that can rotate within the body to select the correct sequence. The plunger contains a rib extending axially along its length which can engage one of a number of grooves or tracks on the inside of the syringe body so that, once the plunger has been rotated to align with the correct sequence of indicia, it is pressed into the syringe body such that the rib engages with the corresponding groove or track and cannot be rotated further.

It will be appreciated to the person of skill in the art that various modifications may be made to the above described embodiments without departing from the scope of the present invention.

## Claims

1. A multi-dose injection syringe (1) comprising a syringe body (2) for containing a medicine and a plunger moveable within the syringe body, from an injection start position, to eject medicine therefrom, the syringe further comprising a scale provided on one of the syringe body (2) and the plunger and orientated along the path of progression of the plunger,
and **characterised in that** the scale provides a time ordered sequence of indicia and that is configurable to align any one of the indicia with said injection start position, at least part of the syringe body (2) being transparent such that the plunger's degree of progression through the syringe body, with respect to the time ordered sequence of indicia, is externally visible.

2. A multi-dose injection syringe as claimed in claim 1, wherein the scale comprises a rotatable sleeve (5) positioned around the outside of the syringe body, the rotatable sleeve defining a window (6), and a number of time ordered sequences of indicia with different starting points being located on the syringe body.

3. A multi-dose injection syringe as claimed in claim 2, the scale being such that configuration is performed by rotating the rotatable sleeve to align the window with the time ordered sequence of indicia with the required start point.

4. A multi-dose injection syringe as claimed in claim 1, wherein the scale comprises a number of superposed removable labels (10) located on the outside surface of the syringe body, each superposed label comprising a time ordered sequence of indicia with a different starting point.

5. A multi-dose injection syringe as claimed in claim 4, the scale being such that configuration is performed by peeling off one or more of the superposed removable labels, such that the top label that remains attached to the syringe body shows the time ordered sequence of indicia with the required start point.

6. A multi-dose injection syringe as claimed in claim 1, wherein the scale comprises a removable and replaceable cover (15) that is separable into sections (16) located around the syringe body, the cover containing the time ordered sequence of indicia.

7. A multi-dose injection syringe as claimed in claim 6, the scale being such that configuration is performed by removing the cover from the syringe body, splitting the cover into sections at a point above the desired starting point of the time ordered sequence of indicia, and replacing the cover with the sections in a different order, such that the time ordered sequence of indicia starts at the desired starting point.

8. A multi-dose injection syringe as claimed in claim 6 or 7, wherein the removable and replaceable cover comprises weakening score lines (17) above potential starting points of the time ordered sequence of indicia.

9. A multi-dose injection syringe as claimed in claim 1, wherein the scale comprises a number of time ordered sequences of indicia with different starting points located on a shaped neck portion of the plunger (22), and a window (23) provided in the syringe body such that one of the indicia can be viewed through the window during use of the syringe.

10. A multi-dose injection syringe as claimed in claim 9, the scale being such that configuration is performed by selecting the time ordered sequence of indicia with the desired starting point, and lining it up with the window in the syringe prior to pressing the plunger into the syringe body.

11. A multi-dose injection syringe as claimed in claim 9 or 10, wherein a complementary shaped opening in the syringe body stops the shaped neck portion of the plunger from being rotated once it has been pressed into the syringe body.

12. A multi-dose injection syringe as claimed in claim 1, wherein the scale comprises a number of strips (30) removably attached along the length of the syringe body, each strip covering a time ordered sequence of indicia with different starting points located on the syringe body.

13. A multi-dose injection syringe as claimed in claim 12, the scale being such that configuration is performed by removing the strip (32) covering the desired time ordered sequence of indicia.

14. A multi-dose injection syringe as claimed in claim 1, wherein the scale comprises a number of sleeves (35), each one provided with a time ordered sequence of indicia with different starting points.

15. A multi-dose injection syringe as claimed in claim 14, the scale being such that configuration is performed by selecting one of the number of sleeves that contains the time ordered sequence of indicia with the desired starting point, and sliding the sleeve over the syringe body.

## Patentansprüche

1. Mehrfachdosierungsinjektionsspritze (1), die einen Spritzenkörper (2) zum Aufnehmen eines Medikaments und einen Tauchkolben, der innerhalb des Spritzenkörpers von einer Injektionsstartposition aus beweglich ist, um ein Medikament daraus auszustoßen, umfasst, wobei die Spritze ferner eine Skala umfasst, die auf einem von dem Spritzenkörper (2) und dem Tauchkolben bereitgestellt wird und entlang der Bahn des Fortschreitens des Tauchkolbens ausgerichtet ist,
und **dadurch gekennzeichnet, dass** die Skala eine zeitlich geordnete Folge von Kennzeichen bereitstellt und die konfigurierbar ist, um ein beliebiges der Kennzeichen mit der Injektionsstartposition auszurichten, wobei wenigstens ein Teil des Spritzenkörpers (2) transparent ist, so dass der Grad des Fortschreitens des Tauchkolbens durch den Spritzenkörper, in Bezug auf die zeitlich geordnete Folge von Kennzeichen, äußerlich sichtbar ist.

2. Mehrfachdosierungsinjektionsspritze nach Anspruch 1, wobei die Skala eine drehbare Hülse (5) umfasst, die um die Außenseite des Spritzenkörpers angeordnet ist, wobei die drehbare Hülse ein Fenster (6) definiert und eine Anzahl von zeitlich geordneten Folgen von Kennzeichen mit unterschiedlichen Startpunkten auf dem Spritzenkörper angeordnet ist.

3. Mehrfachdosierungsinjektionsspritze nach Anspruch 2, wobei die Skala derart ist, dass die Konfiguration ausgeführt wird durch das Drehen der drehbaren Hülse, um das Fenster mit der zeitlich geordneten Folge von Kennzeichen mit dem erforderlichen Startpunkt auszurichten.

4. Mehrfachdosierungsinjektionsspritze nach Anspruch 1, wobei die Skala eine Anzahl von übereinanderliegenden abnehmbaren Etiketten (10), die auf der Außenfläche des Spritzenkörpers angeordnet sind, wobei jedes übereinanderliegende Etikett eine zeitlich geordnete Folge von Kennzeichen mit einem unterschiedlichen Startpunkt umfasst.

5. Mehrfachdosierungsinjektionsspritze nach Anspruch 4, wobei die Skala derart ist, dass die Konfiguration ausgeführt wird durch das Abziehen eines oder mehrerer der übereinanderliegenden abnehmbaren Etiketten, so dass das oberste Etikett, das an dem Spritzenkörper befestigt bleibt, die zeitlich geordnete Folge von Kennzeichen mit dem erforderlichen Startpunkt zeigt.

6. Mehrfachdosierungsinjektionsspritze nach Anspruch 1, wobei die Skala eine abnehmbare und wiederanbringbare Abdeckung (15) umfasst, die in Sektionen (16) trennbar ist, die um den Spritzenkörper angeordnet sind, wobei die Abdeckung die zeitlich geordnete Folge von Kennzeichen enthält.

7. Mehrfachdosierungsinjektionsspritze nach Anspruch 6, wobei die Skala derart ist, dass die Konfiguration ausgeführt wird durch das Entfernen der Abdeckung von dem Spritzenkörper, das Spalten der Abdeckung in Sektionen an einem Punkt oberhalb des gewünschten Startpunkts der zeitlich geordneten Folge von Kennzeichen und das Wiederanbringen der Abdeckung mit den Sektionen in einer anderen Reihenfolge, so dass die zeitlich geordnete Folge von Kennzeichen an dem gewünschten Startpunkt beginnt.

8. Mehrfachdosierungsinjektionsspritze nach Anspruch 6 oder 7, wobei die abnehmbare und wiederanbringbare Abdeckung schwächende Kerblinien (17) oberhalb möglicher Startpunkte der zeitlich geordneten Folge von Kennzeichen umfasst.

9. Mehrfachdosierungsinjektionsspritze nach Anspruch 1, wobei die Skala eine Anzahl von zeitlich geordneten Folgen von Kennzeichen mit unterschiedlichen Startpunkten, angeordnet auf einem geformten Halsabschnitt des Tauchkolbens (22), und ein Fenster (23), das in dem Spritzenkörper bereitgestellt wird, umfasst, so dass eines der Kennzeichen während der Verwendung der Spritze durch das Fenster betrachtet werden kann.

10. Mehrfachdosierungsinjektionsspritze nach Anspruch 9, wobei die Skala derart ist, dass die Konfiguration ausgeführt wird durch das Auswählen der zeitlich geordneten Folge von Kennzeichen mit dem gewünschten Startpunkt und das Ausrichten mit dem Fenster in der Spritze vor dem Drücken des Tauchkolben in den Spritzenkörper.

11. Mehrfachdosierungsinjektionsspritze nach Anspruch 9 oder 10, wobei eine komplementär geformte Öffnung in dem Spritzenkörper den geformten Halsabschnitt des Tauchkolben daran hindert, gedreht zu werden, sobald er in den Spritzenkörper gedrückt worden ist.

12. Mehrfachdosierungsinjektionsspritze nach Anspruch 1, wobei die Skala eine Anzahl von Streifen (30) umfasst, die abnehmbar entlang der Länge des Spritzenkörpers befestigt sind, wobei jeder Streifen eine zeitlich geordnete Folge von Kennzeichen mit unterschiedlichen Startpunkten abdeckt, die auf dem Spritzenkörper angeordnet sind.

13. Mehrfachdosierungsinjektionsspritze nach Anspruch 12, wobei die Skala derart ist, dass die Konfiguration ausgeführt wird durch das Entfernen des Streifens (32), der die zeitlich geordnete Folge von Kennzeichen abdeckt.

14. Mehrfachdosierungsinjektionsspritze nach Anspruch 1, wobei die Skala eine Anzahl von Hülsen (35) umfasst, deren jede mit einer zeitlich geordneten Folge von Kennzeichen mit unterschiedlichen Startpunkten versehen ist.

15. Mehrfachdosierungsinjektionsspritze nach Anspruch 14, wobei die Skala derart ist, dass die Konfiguration ausgeführt wird durch das Auswählen einer von der Anzahl von Hülsen, welche die zeitlich geordnete Folge von Kennzeichen mit dem gewünschten Startpunkt enthält, und das Schieben der Hülse über den Spritzenkörper.

## Revendications

1. Seringue d'injection de doses multiples (1) comprenant un corps de seringue (2) permettant de contenir un médicament et un piston mobile à l'intérieur du corps de seringue, depuis une position de démarrage d'injection, pour éjecter un médicament hors de celui-ci, la seringue comprenant en outre une graduation prévue sur l'un parmi le corps de seringue (2) et le piston et orientée le long de la trajectoire de progression du piston,
et **caractérisée en ce que** la graduation prévoit une séquence d'indices ordonnée dans le temps et qui est configurable pour aligner l'un quelconque des indices avec ladite position de démarrage d'injection, au moins une partie du corps de seringue (2) étant transparente de telle sorte que le degré de progression du piston à travers le corps de seringue, par rapport à la séquence d'indices ordonnée dans le temps, est visible depuis l'extérieur.

2. Seringue d'injection de doses multiples selon la revendication 1, dans laquelle la graduation comprend un manchon rotatif (5) positionné autour de l'extérieur du corps de seringue, le manchon rotatif définissant une fenêtre (6), et une pluralité de séquences d'indices ordonnées dans le temps avec différents points de démarrage qui sont situés sur le corps de seringue.

3. Seringue d'injection de doses multiples selon la revendication 2, la graduation étant telle qu'une configuration est effectuée par une rotation du manchon rotatif pour aligner la fenêtre avec la séquence d'indices ordonnée dans le temps avec le point de démarrage requis.

4. Seringue d'injection de doses multiples selon la revendication 1, dans laquelle la graduation comprend une pluralité d'étiquettes amovibles superposées (10) situées sur la surface extérieure du corps de seringue, chaque étiquette superposée comprenant une séquence d'indices ordonnée dans le temps avec un point de démarrage différent.

5. Seringue d'injection de doses multiples selon la revendication 4, la graduation étant telle qu'une configuration est effectuée en arrachant une ou plusieurs des étiquettes amovibles superposées, de telle sorte que l'étiquette supérieure qui reste attachée au corps de seringue montre la séquence d'indices ordonnée dans le temps avec le point de démarrage requis.

6. Seringue d'injection de doses multiples selon la revendication 1, dans laquelle la graduation comprend une enveloppe amovible et remplaçable (15) qui est séparable en sections (16) situées autour du corps de seringue, l'enveloppe contenant la séquence d'indices ordonnée dans le temps.

7. Seringue d'injection de doses multiples selon la revendication 6, la graduation étant telle qu'une configuration est effectuée en retirant l'enveloppe du corps de seringue, en divisant l'enveloppe en sections au niveau d'un point au-dessus du point de démarrage souhaité de la séquence d'indices ordonnée dans le temps, et en remplaçant l'enveloppe avec les sections dans un ordre différent, de telle sorte que la séquence d'indices ordonnée dans le temps démarre au niveau du point de démarrage souhaité.

8. Seringue d'injection de doses multiples selon la revendication 6 ou 7, dans laquelle l'enveloppe amovible et remplaçable comprend des lignes de repère d'affaiblissement (17) au-dessus de points de démarrage potentiels de la séquence d'indices ordonnée dans le temps.

9. Seringue d'injection de doses multiples selon la revendication 1, dans laquelle la graduation comprend une pluralité de séquences d'indices ordonnées dans le temps avec différents points de démarrage situés sur une partie formant encolure façonnée du piston (22), et une fenêtre (23) prévue dans le corps de seringue de telle sorte que l'un des indices peut être visualisé à travers la fenêtre pendant l'utilisation de la seringue.

10. Seringue d'injection de doses multiples selon la revendication 9, la graduation étant telle qu'une configuration est effectuée en sélectionnant la séquence d'indices ordonnée dans le temps avec le point de démarrage souhaité, et en l'alignant avec la fenêtre dans la seringue avant de pousser le piston dans le corps de seringue.

11. Seringue d'injection de doses multiples selon la revendication 9 ou 10, dans laquelle une ouverture façonnée complémentaire dans le corps de seringue arrête la rotation de la partie formant encolure façonnée du piston une fois que celui-ci a été poussé dans le corps de seringue.

12. Seringue d'injection de doses multiples selon la revendication 1, dans laquelle la graduation comprend une pluralité de bandes (30) attachées de manière amovible sur la longueur du corps de seringue, chaque bande couvrant une séquence d'indices ordonnée dans le temps avec différents points de démarrage situés sur le corps de seringue.

13. Seringue d'injection de doses multiples selon la revendication 12, la graduation étant telle qu'une configuration est effectuée en retirant la bande (32) couvrant la séquence d'indices ordonnée dans le temps souhaitée.

14. Seringue d'injection de doses multiples selon la revendication 1, dans laquelle la graduation comprend une pluralité de manchons (35), chacun étant pourvu d'une séquence d'indices ordonnée dans le temps avec différents points de démarrage.

15. Seringue d'injection de doses multiples selon la revendication 14, la graduation étant telle qu'une configuration est effectuée en sélectionnant l'un d'une pluralité de manchons qui contient la séquence d'indices ordonnée dans le temps avec le point de démarrage souhaité, et en faisant glisser le manchon sur le corps de seringue.
